(19) Europäisches Patentamt
European Patent Office
Office européen des brevets

(11) **EP 4 174 163 A1**

(12) **EUROPEAN PATENT APPLICATION**
published in accordance with Art. 153(4) EPC

(43) Date of publication:
**03.05.2023 Bulletin 2023/18**

(21) Application number: **21849563.8**

(22) Date of filing: **28.07.2021**

(51) International Patent Classification (IPC):
***C12M 1/00*** *(2006.01)*   ***C12M 3/00*** *(2006.01)*
***C12N 5/071*** *(2010.01)*   ***C12N 5/073*** *(2010.01)*

(52) Cooperative Patent Classification (CPC):
**C12M 1/00; C12M 3/00; C12N 5/06**

(86) International application number:
**PCT/JP2021/027820**

(87) International publication number:
**WO 2022/025092 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.07.2020   JP 2020127635
27.07.2021   JP 2021122191**

(71) Applicant: **University of Yamanashi
Kofu-shi, Yamanashi 400-8510 (JP)**

(72) Inventors:
• **WAKAYAMA, Teruhiko
Kofu-shi, Yamanashi 400-8510 (JP)**
• **WAKAYAMA, Sayaka
Kofu-shi, Yamanashi 400-8510 (JP)**
• **SUZUKI, Tomomi
Chofu-shi, Tokyo 182-8522 (JP)**
• **YAMAZAKI, Chiaki
Chofu-shi, Tokyo 182-8522 (JP)**

(74) Representative: **Richardt Patentanwälte PartG
mbB
Wilhelmstraße 7
65185 Wiesbaden (DE)**

(54) **FROZEN EGG CULTIVATION APPARATUS, AND METHOD FOR CULTIVATING FROZEN EGGS**

(57)   A frozen egg culturing device includes: a housing portion configured to house a frozen egg contained in a container; a liquid injecting portion; a liquid discharging portion; and an egg outflow preventing portion between the frozen egg contained in the container, and the liquid injecting portion and the liquid discharging portion. A frozen egg culturing method uses the frozen egg culturing device.

EP 4 174 163 A1

**Description**

[Technical Field]

The present invention relates to a frozen egg culturing device or method of culturing a frozen egg (a frozen fertilized or unfertilized egg).

[Background Art]

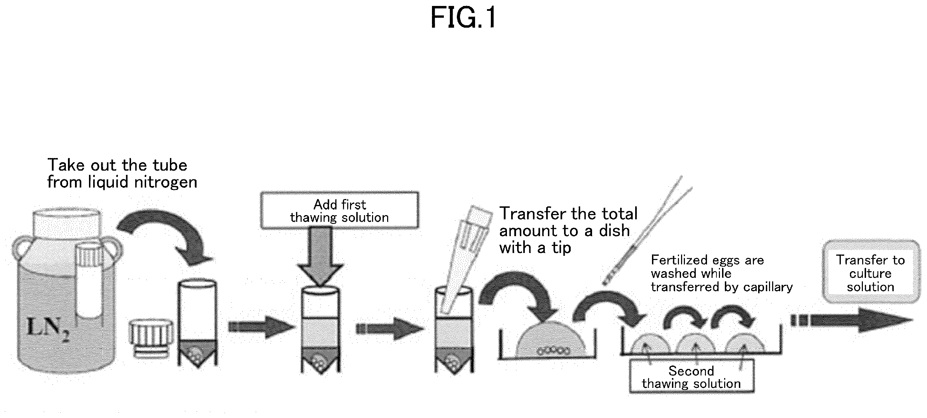

Conventionally, frozen eggs of mammals such as a mouse have been thawed, washed, and cultured in an open system as illustrated in FIG. 1. Specifically, a frozen egg contained in a container such as a cryotube is stored in liquid nitrogen, and the stored frozen egg is thawed and washed as follows. First, a first thawing solution is added to the container such as the cryotube, then the total amount of the resulting mixture is transferred to a culture dish by pipetting, and then eggs (fertilized or unfertilized eggs) are washed while sequentially transferred by a capillary tube to three drops of a second thawing solution that are separately disposed on the culture dish. The washed eggs are transferred to a culture solution and are cultured. This operation of handling small eggs having a size of about 100 $\mu$m can be successfully performed only by skilled technicians, and this is not an operation that anyone can do successfully.

The technique of thawing, washing, and culturing a frozen egg is used in various fields such as clinics for fertility treatments, facilities for animal experiments, and animal husbandry. Thus, if it is possible to develop a technique that enables anyone who is not a skilled technician to readily and surely thaw, wash, and culture a frozen egg, such a technique can make a great contribution to developments in the above fields.

To date, proposed is a germ cell cryopreservation method in which germ cells are contained in a hollow fiber and frozen (see, for example, PTL 1). In the above proposal, it is described that germ cells can be cryopreserved in a simple manner. However, the above proposed device is one that is configured to cryopreserve germ cells, not one that is configured to thaw, wash, and culture germ cells in the same device.

Also, as a technique of allowing for readily recovering valuable biological cells leaked from a freezing container without any risk of inhibiting heat conduction during de-freezing of frozen cells, proposed is a package that is provided with an exhaust port through which the air inside the package is to be discharged, and the package covers the freezing container that contains frozen cells (see, for example, PTL 2). However, the above proposed package is a bag that is configured to defreeze frozen cells, not one that is configured to wash and culture the de-frozen cells in the same container.

Also, proposed is a kit of preparation of antigen-specific cytotoxic T cells, where the kit includes, as a constituting component, a sealable culture container having a plurality of ports (see, for example, PTL 3). In the above proposal, it is described that antigen-specific cytotoxic T cells can be prepared by a simple operation. However, the above proposal is for the preparation of antigen-specific cytotoxic T cells, and is not about a technique of thawing, washing, and culturing a frozen egg.

At present, therefore, there has been no technique that enables anyone as well as skilled technicians to thaw, wash, and culture a frozen egg.

In addition, understanding of life phenomena and the possibility of reproduction in space has been a theme of great interest since the beginning of the development of rockets. In recent years, attention has been attracted to the early life stage of mammals in a space environment. In space, however, experiments to culture fertilized eggs have not yet been realized. This is because there is zero gravity in space, and embryos cannot be manipulated like on the ground. This is also because there are conditional constraints such as the inability to use liquid nitrogen, and technical constraints such as the difficulty of astronauts performing operations such as thawing, washing, and culturing a frozen egg, which require high skills.

Therefore, from the viewpoint of making it possible to conduct experiments in space, there is a strong demand for the development of a technique that enables anyone as well as skilled technicians to thaw, wash, and culture a frozen egg.

[Citation List]

[Patent Literature]

[PTL 1] Japanese Patent Application Laid-Open No. 2009-148218
[PTL 2] Japanese Patent Application Laid-Open No. 2001-070402
[PTL 3] Japanese Patent Application Laid-Open No. 2010-130999

[Summary of Invention]

[Technical Problem]

The present invention aims to meet such demands, overcome the current circumstances, solve the conventional problems, and achieve the following object. Specifically, the present invention has an object to provide a frozen egg culturing device or method that enables anyone as well as skilled technicians to thaw, wash, and culture a frozen egg.

[Solution to Problem]

The present inventors conducted intensive studies to achieve the above object, and have found that, by providing a frozen egg culturing device including: a housing portion configured to house a frozen egg contained in a container; a liquid injecting portion; a liquid discharging portion; and an egg outflow preventing portion between the frozen egg contained in the container, and the liquid injecting portion and the liquid discharging portion, it is possible to thaw, wash, and culture the frozen egg in a sealed container (a closed system; i.e., a system separated from the outside air) without touching the frozen egg, and it is therefore possible for even a beginner, who has never handled a fertilized or unfertilized egg, to thaw, wash, and culture the frozen egg without receiving any training.
The present invention is based on the above finding obtained by the present inventors, and means for achieving the object are as follows.

<1> A frozen egg culturing device, including:

a housing portion configured to house a frozen egg contained in a container;
a liquid injecting portion;
a liquid discharging portion; and
an egg outflow preventing portion,
the egg outflow preventing portion being between

the frozen egg contained in the container, and
the liquid injecting portion and the liquid discharging portion.

<2> A frozen egg culturing method, including:
using the frozen egg culturing device according to <1> above.

[Advantageous Effects of Invention]

The present invention can solve the above-described various conventional problems and achieve the above object, and can provide a frozen egg culturing device or method that enables anyone as well as skilled technicians to thaw, wash, and culture a frozen egg.

[Brief Description of Drawings]

[FIG. 1] FIG. 1 is a view illustrating a conventional method of thawing and washing frozen eggs.
[FIG. 2A] FIG. 2A is a schematic view illustrating one side of one example of a frozen egg culturing device.
[FIG. 2B] FIG. 2B is a schematic view illustrating the other side of the frozen egg culturing device of FIG. 2A.
[FIG. 2C] FIG. 2C is a photograph of the frozen egg culturing device of FIG. 2A.
[FIG. 3] FIG. 3 is a photograph of one example of a frozen egg storing container.
[FIG. 4] FIG. 4 is a view illustrating one example of a state where syringes and the like are connected to a frozen egg culturing device.
[FIG. 5] FIG. 5 is a view illustrating frozen fertilized eggs cultured using a frozen egg culturing device of the present invention.
[FIG. 6] FIG. 6 is a view illustrating one example of a frozen egg storing container.
[FIG. 7A] FIG. 7A is a view illustrating one example of frozen fertilized eggs cultured using a frozen egg culturing device having no adhesion preventing unit of Example 2-1 (many of the developed embryos are deformed).
[FIG. 7B] FIG. 7B is a view illustrating one example of frozen fertilized eggs cultured using a frozen egg culturing device having an adhesion preventing unit of Example 2-2 (many of the developed embryos are kept to have a round shape).

[FIG. 8A] FIG. 8A is a schematic view illustrating a cross section of a housing portion that is assumed upon discharge of, for example, a culture solution when using a frozen egg culturing device having no adhesion preventing unit.

[FIG. 8B] FIG. 8B is a schematic view illustrating a cross section of a housing portion that is assumed upon discharge of, for example, a culture solution when using a frozen egg culturing device having an adhesion preventing unit.

[Description of Embodiments]

(Frozen egg culturing device)

The frozen egg culturing device of the present invention includes: a housing portion configured to house a frozen egg contained in a container; a liquid injecting portion; a liquid discharging portion; and an egg (a fertilized or unfertilized egg) outflow preventing portion. If necessary, the frozen egg culturing device of the present invention further includes other components.

<Frozen egg>

In the present invention, the frozen egg refers to a frozen fertilized or unfertilized egg. Also, the fertilized egg includes not only eggs immediately after fertilization but also early embryos. Culturing the frozen egg means de-freezing (thawing) the frozen egg and culturing the resulting egg.

A species of the frozen egg is not particularly limited and may be appropriately selected in accordance with the intended purpose. Mammals are preferable. The mammals are not particularly limited and may be appropriately selected in accordance with the intended purpose. Examples of the mammals include humans, mice, rats, rabbits, dogs, cats, horses, cattle, pigs, goats, sheep, and marine mammals.

A preparation method of the frozen egg is not particularly limited and may be appropriately selected from publicly known methods.

<Housing portion configured to house a frozen egg contained in a container>

The housing portion is configured to house a frozen egg contained in a container.

The shape, structure, and size of the housing portion are not particularly limited and may be appropriately selected in accordance with the intended purpose.

-Frozen egg contained in a container-

The frozen egg is housed in a container configured to house a frozen egg (hereinafter may be referred to as a "frozen egg storing container").

The frozen egg storing container is not particularly limited and may be an appropriately selected publicly known container for storing a frozen egg. Examples of the frozen egg storing container include cryotubes, hollow straws, and processed products thereof. These may be used alone or in combination.

The shape, structure, and size of the frozen egg storing container are not particularly limited and may be appropriately selected in accordance with, for example, the size of the housing portion and the number of frozen eggs contained in containers that are to be housed in the housing portion.

The depth of the frozen egg storing container is not particularly limited and may be appropriately selected in accordance with the intended purpose. From the viewpoint of readily removing a freezing liquid or a thawing solution at the time of de-freezing or washing of the frozen egg, the depth of the frozen egg storing container is preferably from 5 mm to 15 mm. The above depth means the length of the deepest portion therein.

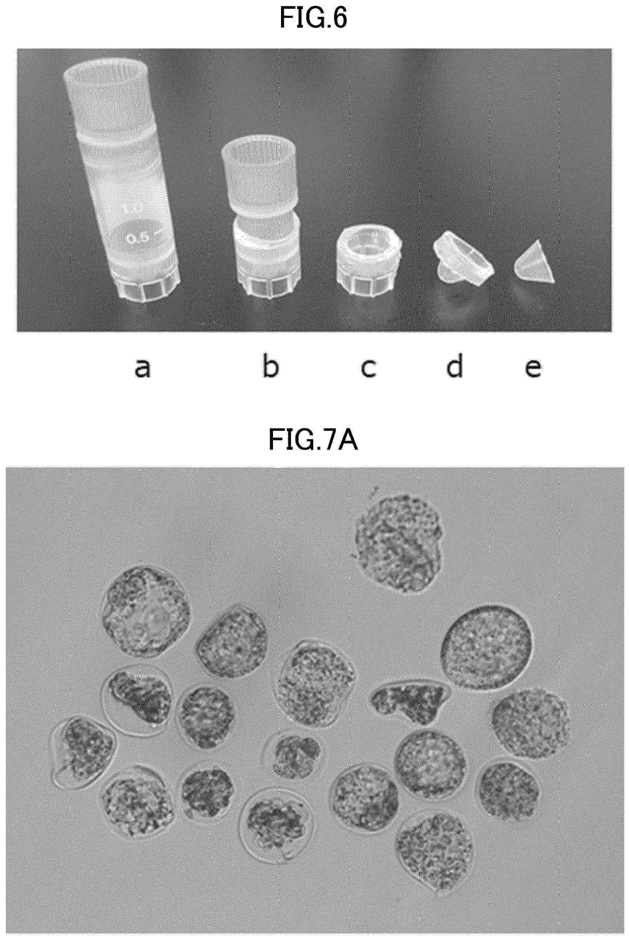

When a cryotube is used as the frozen egg storing container, examples of the shape of the frozen egg storing container include those exemplified in FIG. 6. In FIG. 6, "a" is the shape of the cryotube itself, "b" is the shape of a product obtained by cutting an upper part of the cryotube, "c" is the shape of a product formed from: a bottom part of the cryotube, the bottom part having a shape of letter V in a front view thereof; and a skirt part around the bottom part (hereinafter may be referred to as "M form"), "d" is the shape of a product obtained by removing the skirt part of the "M form" (hereinafter may be referred to as "top form"), and "e" is the shape consisting of a bottom part of the cryotube, the bottom part having a shape of letter V in a front view thereof (hereinafter may be referred to as "V form"). Of these, the shape of letter V is preferable from the viewpoint of readily removing a freezing liquid or a thawing solution at the time of de-freezing or washing of the frozen egg and increasing the recovery rate of blastocysts.

The shape in a front view refers to the shape of a cross section formed by vertically cutting the frozen egg storing container that is placed on a flat plane, with the open part of the frozen egg storing container facing upward.

The material of the frozen egg storing container is not particularly limited and may be appropriately selected in accordance with the intended purpose, as long as the material does not impair the effects of the present invention. Examples of the material include polypropylene and polyethylene.

A producing method of the frozen egg storing container is not particularly limited and may be produced by a publicly known unit that is appropriately selected.

<Liquid injecting portion>

The liquid injecting portion is for injecting a liquid into the housing portion.

The position of the liquid injecting portion is not particularly limited and may be appropriately selected in accordance with the intended purpose. The liquid injecting portion is preferably provided, for example, at the end of the frozen egg culturing device.

The number of liquid injecting portions may be one or may be two or more. From the viewpoint of readily handling, the number of liquid injecting portions is preferably one.

<Liquid discharging portion>

The liquid discharging portion is for discharging a liquid in the housing portion.

The position of the liquid discharging portion is not particularly limited and may be appropriately selected in accordance with the intended purpose. The liquid discharging portion is preferably provided at the same end of the frozen egg culturing device at which the liquid injecting portion is provided.

The number of liquid discharging portions may be one or may be two or more. From the viewpoint of readily handling, the number of liquid discharging portions is preferably one.

The interval between the position of the liquid injecting portion and the position of the liquid discharging portion is not particularly limited and may be appropriately selected in accordance with the intended purpose.

The forms of the liquid injecting portion and the liquid discharging portion are not particularly limited and may be appropriately selected in accordance with the intended purpose. Examples of preferable forms of the liquid injecting portion and the liquid discharging portion include those in which the below-described liquid injecting unit and liquid discharging unit can be connected to the liquid injecting portion and the liquid discharging portion, respectively; e.g., those of a luer lock type, needle sticking ports, and membrane tubes.

The ends of the liquid injecting portion and the liquid discharging portion may be provided with a member such as a cap, in order to avoid contact with the outside.

<Egg outflow preventing portion>

The egg outflow preventing portion is for preventing outflow of an egg (a fertilized or unfertilized egg).

The placement of the egg outflow preventing portion is not particularly limited and may be appropriately selected in accordance with the intended purpose, as long as the egg outflow preventing portion is between the frozen egg contained in the container, and the liquid injecting portion and the liquid discharging portion.

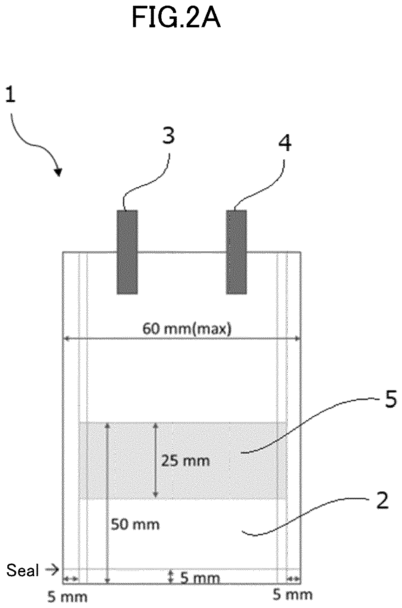

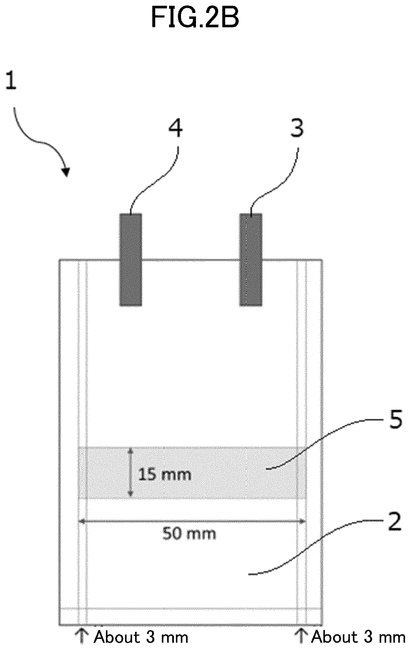

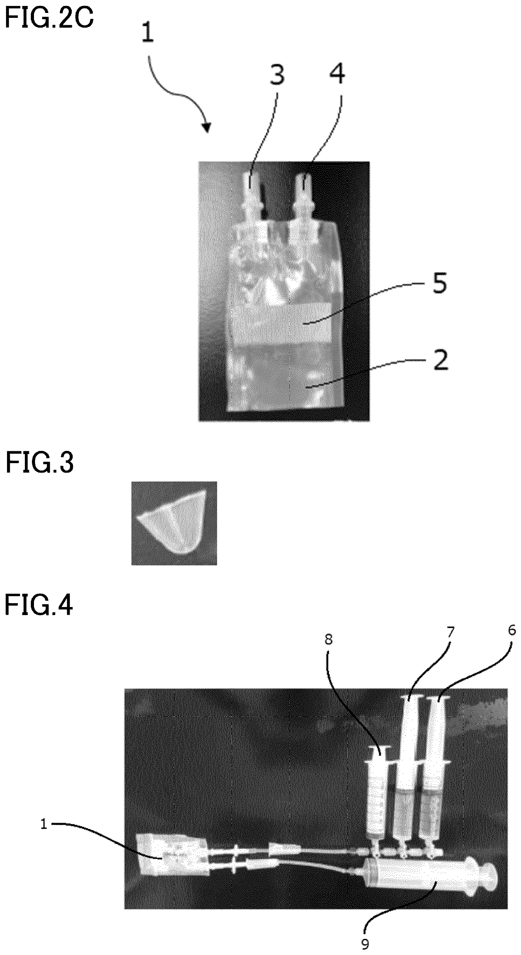

Specific examples of the manner in which the egg outflow preventing portion is placed include: a manner in which the egg outflow preventing portion is placed to cover the openings (inlets/outlets) on the housing portion side of the liquid injecting portion and the liquid discharging portion; a manner in which the egg outflow preventing portion is placed in the housing portion in the form of a bag configured to house the frozen egg contained in the container; and a manner in which the egg outflow preventing portion is placed in the housing portion and partitions the side of the housing portion at which the frozen egg contained in the container is placed and the side of the liquid injecting portion and the liquid discharging portion (i.e., a manner in which the egg outflow preventing portion is placed in the form of a wall so as to divide the side of the housing portion in which the frozen egg contained in the container is placed and the side of the end provided with the liquid injecting portion and the liquid discharging portion (see, for example, FIG. 2A and FIG. 2B)).

In the manner in which the egg outflow preventing portion is placed in the form of a wall, a member for forming the egg outflow preventing portion is folded in two, and the folded member is placed such that the open side thereof faces the side at which the frozen egg contained in the container is placed.

The size of the egg outflow preventing portion is not particularly limited and may be appropriately selected in accordance with, for example, the size of the container that houses the frozen egg and the size of the housing portion.

When the egg outflow preventing portion is placed in the form of a wall, the egg outflow preventing portion is placed from one end of the housing portion to the other end thereof.

The material of the egg outflow preventing portion is not particularly limited and may be appropriately selected in accordance with the intended purpose, as long as the material is low-toxic to the egg. Examples of the material include nylon. These may be used alone or in combination.

The structure of the egg outflow preventing portion is not particularly limited and may be appropriately selected in accordance with the intended purpose, as long as the structure does not allow permeation of an egg and allows permeation of liquids such as a freezing liquid, a thawing solution, and a washing liquid. Examples of the structure include a mesh structure.

The opening of the mesh is not particularly limited and may be appropriately selected in accordance with, for example, the size of the egg, as long as the opening does not allow permeation of an egg and allows permeation of liquids such as a freezing liquid. For an egg of a mouse, the opening is, for example, 40 $\mu$m. The egg outflow preventing portion may be a single layer or a multilayer of two or more layers.

<Other components>

The other components are not particularly limited and may be appropriately selected in accordance with the intended purpose, as long as the other components do not impair the effects of the present invention. Examples of the other components include an adhesion preventing unit and a cooling container.

<<Adhesion preventing unit>>

The adhesion preventing unit is configured to prevent adhesion between the top face and the bottom face of the housing portion when discharging the liquid in the housing portion. By providing the housing portion with the adhesion preventing unit, it is possible to form (maintain) a space between the top face and the bottom face of the housing portion when discharging the liquid in the housing portion. This makes it possible to increase the recovery rate of embryos and reduce deformation of embryos. Therefore, the frozen egg culturing device preferably includes the adhesion preventing unit in a region of the housing portion where the frozen egg contained in the container is to be placed. Note that, the adhesion preventing unit may be referred to as a space maintaining unit or a collapse preventing unit. The space between the top face and the bottom face of the housing portion is not particularly limited and may be appropriately selected in accordance with the intended purpose. The length (thickness) between the top face and the bottom face of the housing portion when discharging the liquid in the housing portion is preferably about 2 mm to about 7 mm.

The adhesion preventing unit may be one or two or more. The shape, structure, and size of the adhesion preventing unit are not particularly limited and appropriately selected in consideration of the thickness between the top face and the bottom face.

The position at which the adhesion preventing unit is to be placed is not particularly limited and may be appropriately selected in accordance with the intended purpose. When two or more adhesion preventing units as described above are used, for example, the adhesion preventing units are arranged at the corners of or at the corners and in a central area of a region of the housing portion where the frozen egg contained in the container is to be placed. The two or more adhesion preventing units may or may not be arranged at equal intervals.

The adhesion preventing units may or may not be fixed at sites at which the adhesion preventing units are disposed.

The adhesion preventing unit that is to be fixed may be provided in advance as a partition wall in a region of the housing portion where the frozen egg contained in the container is to be placed.

The shape of the adhesion preventing unit that is not to be fixed is not particularly limited and may be appropriately selected in accordance with the intended purpose. Examples of the shape include planar shapes such as a plate shape, and three-dimensional shapes such as a curved plate shape, a spherical shape, and a tubular shape. Of these, from the viewpoint of increasing the recovery rate of embryos and reducing deformation of embryos, three-dimensional shapes are preferable, with a curved plate shape or a spherical shape being more preferable.

The size of the adhesion preventing unit that is not to be fixed is not particularly limited and may be appropriately selected in accordance with the intended purpose. From the viewpoint of reducing scratch of an egg, the adhesion preventing unit preferably has a size that is larger than 1 mm×1 mm, and more preferably has a size of about 3 mm in width×about 10 mm in length.

It is preferable that the adhesion preventing units not to be fixed be uniformly dispersed to exist in a region of the housing portion where the frozen eggs contained in the container are to be placed.

The area of the adhesion preventing units relative to the region of the housing portion where the frozen eggs contained in the containers are to be placed is not particularly limited and may be appropriately selected in accordance with the intended purpose. The above area is, for example, from about 30% to about 50%. The area of the adhesion preventing units relative to the region of the housing portion where the frozen eggs contained in the containers are to be placed refers to an area in a plan view of the frozen egg culturing device that is placed on a flat surface.

The adhesion preventing unit is preferably subjected to a chamfering process, a surface smoothing process, etc. in order to prevent possible damage to the embryos or device.

The material of the adhesion preventing unit is not particularly limited and may be appropriately selected in accordance with the intended purpose, as long as the material is not toxic to the embryos.

<<Cooling container>>

By using the cooling container, it is possible to prevent a rapid change in temperature until a thawing solution is poured to the frozen eggs in the frozen egg culturing device. As a result, it is possible to increase the survival rate of the frozen eggs.

The size of the cooling container is not particularly limited and may be appropriately selected in accordance with, for example, the size of the frozen egg culturing device.

The shape of the cooling container is not particularly limited and may be appropriately selected in accordance with the intended purpose. Examples of the shape include the shape of a bag. When the cooling container is formed into a bag that is large enough to house the whole frozen egg culturing device, it is possible to more effectively prevent a rapid change in temperature.

The material of the cooling container is not particularly limited and may be appropriately selected in accordance with the intended purpose, as long as the material is usable in liquid nitrogen. Examples of the material include a nylon mesh and small aluminum particles.

The structure of the cooling container is not particularly limited and may be appropriately selected in accordance with the intended purpose, as long as the structure is usable in liquid nitrogen. Examples of the structure include a structure where small aluminum particles are contained in, for example, a bag made of a mesh material such as a nylon mesh.

<Producing method of the frozen egg culturing device>

A producing method of the frozen egg culturing device is not particularly limited. The frozen egg culturing device can be produced from appropriately selected publicly known materials using a publicly known method so as to have the above structure. For example, the frozen egg culturing device can be produced by, for example, attaching an egg outflow preventing portion to a container that can be cryopreserved, such as FROZEBAG (NIPRO CORPORATION).

The shape and size of the frozen egg culturing device are not particularly limited and may be appropriately selected in accordance with the intended purpose.

The structure of the frozen egg culturing device is not particularly limited and may be appropriately selected in accordance with the intended purpose, as long as the structure does not adversely affect the eggs contained therein. For example, the structure of the frozen egg culturing device may or may not have gas permeability.

When the frozen egg culturing device has gas permeability, culturing can be performed using a publicly known incubator for culturing embryos, such as a $CO_2$ incubator.

When the frozen egg culturing device has no gas permeability, culturing can be performed anywhere as long as the temperature is controlled. This is because the frozen egg culturing device having no gas permeability is a hermetically sealed container (a closed system). Note that, "hermetically sealed" means being hermetically sealed at the time of culturing.

In the present invention, "no gas permeability" means 5 mL/m$^2$/day/MPa or lower as the oxygen gas permeability of an outer member forming the frozen egg culturing device (e.g., when the frozen egg culturing device is a bag, the above outer member is a material forming the bag). In the present invention, the oxygen gas permeability is a value determined at 25°C and 80%RH according to the determination of gas permeability of plastic films and sheets in compliance with JIS K 7126-2.

A member having no gas permeability is not particularly limited and may be appropriately selected from publicly known members in accordance with the intended purpose, as long as the member does not impair the effects of the present invention. The member is preferably a cold-resistant synthetic resin that can be resistant to a low temperature. Examples of such a resin include ultrahigh-molecular-weight polyethylene, ethylene-vinyl acetate copolymers, fluororesins, and polyimide.

The frozen egg culturing device of the present invention enables the frozen egg to be thawed, washed, and cultured after storage in dry ice rather than liquid nitrogen. As a result, it takes less effort to handle, e.g., transport, the frozen eggs.

(Frozen egg culturing method)

The frozen egg culturing method of the present invention is not particularly limited and may be appropriately selected in accordance with the intended purpose, as long as the frozen egg culturing method uses the frozen egg culturing device of the present invention. The frozen egg culturing method includes a thawing step, a washing step, and a culturing step. If necessary, the frozen egg culturing method preferably includes other steps such as a fertilizing step, a freezing step, a recovering step, and a re-freezing step of freezing embryos after culturing.

<Thawing step>

The thawing step is thawing the frozen egg in the frozen egg culturing device.

A thawing solution used in the thawing step is not particularly limited and may be appropriately selected from those used for publicly known thawing methods of frozen eggs. In an exemplary manner, the frozen egg is thawed using a first thawing solution that has a high osmotic pressure and a second thawing solution that has a low osmotic pressure.

<Washing step>

The washing step is washing a thawed egg (a fertilized or unfertilized egg) after the thawing step.

A washing liquid used in the washing step is not particularly limited and may be appropriately selected from those used for publicly known washing methods of eggs. Examples of the washing liquid include a culturing liquid for culturing used in the below-described culturing step and a culturing liquid for fertilizing used in the below-described fertilizing step.

The kind of each of the culturing liquids is not particularly limited and may be appropriately selected from publicly known media that can be used for culturing fertilized eggs or for fertilizing unfertilized eggs. Examples of the culturing liquid include the CZB medium.

When the frozen egg culturing device has gas permeability, each of the culturing liquids can be used in a manner that the culturing liquids are routinely used.

When the frozen egg culturing device has no gas permeability, each of the culturing liquids is used after the $CO_2$ concentration of the culturing liquids has been optimized.

The $CO_2$ concentration of the $CO_2$ concentration-optimized culture solution is not particularly limited and may be appropriately selected in accordance with the intended purpose, as long as the $CO_2$ concentration-optimized culture solution can culture eggs. The $CO_2$ concentration of the $CO_2$ concentration-optimized culture solution is preferably from 3% to 6% and more preferably from 4% to 5%. One of the above preferable ranges is advantageous in that the developmental rate becomes higher.

A method of optimizing the $CO_2$ concentration of each of the culturing liquids is not particularly limited and may be appropriately selected in accordance with the intended purpose. Examples of the method include leaving each culturing liquid to stand still in a $CO_2$ incubator of 37°C for a certain period of time. The certain period of time is not particularly limited and may be appropriately selected in accordance with the intended purpose. Examples of the certain period of time include 24 hours. Alternatively, for example, each culturing liquid is treated with a carbonic acid gas generator called ANAEROPOUCH for about 4 hours so that the $CO_2$ concentration of each culturing liquid can be adjusted to from 4% to 5%.

The timing at which the $CO_2$ concentration is to be optimized is not particularly limited and may be appropriately selected in accordance with the intended purpose. Examples of the timing include 1 day to 2 days before the washing step.

Methods of the thawing step and the washing step are not particularly limited and may be appropriately selected in accordance with the intended purpose, as long as the methods use the frozen egg culturing device of the present invention.

For example, in order to inject the thawing solution and the washing liquid through the liquid injecting portion of the frozen egg culturing device, the injecting unit such as a syringe is charged with the thawing solution or the washing liquid.

From the viewpoint of simply, successfully, and rapidly performing operations, the injecting unit is preferably attached to a connector of a three-way stopcock having a member configured to connect to the liquid injecting portion, such as a needle-equipped tube.

Also, in order to discharge liquid in the housing portion through the liquid discharging portion of the frozen egg culturing device, the discharging unit such as a syringe is equipped with a member configured to connect the liquid discharging portion with the discharging unit, such as a needle-equipped tube.

Next, the injecting unit is connected to the liquid injecting portion of the frozen egg culturing device that was stored, and the liquid discharging unit is connected to the liquid discharging portion.

Note that, when the frozen egg culturing device was stored in the cooling container, at least the liquid injecting portion and the liquid discharging portion of the frozen egg culturing device are taken out from the cooling container, and the liquid discharging unit is connected to the liquid discharging portion.

Subsequently, the thawing solution is injected to the housing portion through the liquid injecting portion.

Next, the thawing solution is discharged from the housing portion through the liquid discharging portion.

When two or more thawing solutions are used, the above operation is performed for each of the thawing solutions.

When injecting the thawing solution, the total amount of the thawing solution may be injected once. Alternatively, the total amount of the thawing solution may be injected in two or more portions (while repeating injecting and discharging).

When using, as the thawing solution, the first thawing solution having a high osmotic pressure and the second thawing solution having a low osmotic pressure, it is preferable to inject the total amount of the first thawing solution once and inject the total amount of the second thawing solution twice half at a time, from the viewpoint of increasing the developmental rate and the recovery rate of blastocysts.

The amount of the thawing solution to be used is not particularly limited and may be appropriately selected in accordance with the amount of the frozen egg (the amount containing the freezing agent; hereinafter may be referred to as "contents"). It is preferable to adjust the amount of the thawing solution to, for example, an amount that is about three times the contents.

Subsequently, the washing liquid is injected to the housing portion through the liquid injecting portion.

Next, the washing liquid is discharged from the housing portion through the liquid discharging portion.

When injecting the washing liquid, the total amount of the washing liquid may be injected once. Alternatively, the total amount of the washing liquid may be injected in two or more portions (while repeating injecting and discharging). It is preferable to inject the total amount of the washing liquid twice half at a time, from the viewpoint of increasing the developmental rate and the recovery rate of blastocysts.

The washing step is preferably performed at least twice or more from the viewpoint of increasing the developmental rate and the recovery rate of blastocysts. In the present invention, the washing step being performed at least twice or more means that the washing step is performed once and after a certain period of time has passed, the washing step is performed again.

The certain period of time is not particularly limited and may be appropriately selected in accordance with the intended purpose. The certain period of time is preferably from 30 minutes to 3 hours and more preferably from 1 hour to 2 hours. The certain period of time within one of the above preferable ranges is advantageous in that it is possible to better remove the freezing liquid to more increase the developmental rate and the recovery rate of blastocysts.

Conditions such as a temperature during the above certain period of time are not particularly limited and may be appropriately selected in accordance with the intended purpose. One exemplary set of the conditions is at from 20°C to 40°C in the dark.

The amount of the washing liquid to be used is not particularly limited and may be appropriately selected in accordance with the amount of the frozen egg (the amount containing the freezing agent; hereinafter may be referred to as "contents").

The duration and temperature of each of the thawing step and the washing step are not particularly limited and may be appropriately selected in accordance with, for example, the developmental rate and the recovery rate of blastocysts.

fertilizing step>

When a frozen unfertilized egg is used as the frozen egg, the fertilizing step is provided after the washing step and before the below-described culturing step.

The fertilizing step is fertilization by placing sperm in the frozen egg culturing device containing an unfertilized egg.

A method of placing sperm in the frozen egg culturing device is not particularly limited and may be appropriately selected in accordance with the intended purpose. Examples of the method include injecting liquid containing the sperm through the liquid injecting portion of the frozen egg culturing device, in the same manner as in the thawing step and the washing step.

The sperm may be unfrozen sperm (fresh sperm) or may be thawed sperm obtained from frozen sperm.

The amount of the sperm to be used is not particularly limited and may be appropriately selected in accordance with, for example, the quantities of the unfertilized eggs.

The temperature and the duration of the fertilizing step are not particularly limited and may be appropriately selected in accordance with the intended purpose. Examples of the temperature and the duration of the fertilizing step include 37°C for from 4 hours to 6 hours.

By providing the fertilizing step, it is possible to thaw, wash, fertilize, and culture the frozen unfertilized egg using the frozen egg culturing device.

<Culturing step>

The culturing step is culturing a fertilized egg that has been washed in the washing step or a fertilized egg that has been fertilized in the fertilizing step.

The temperature of the culturing is not particularly limited and may be appropriately selected in accordance with the intended purpose. Examples of the temperature include about 37°C.

The duration of the culturing is not particularly limited and may be appropriately selected in accordance with the intended purpose. Examples of the duration include from 3 to 4 days.

In the present invention, when the frozen egg culturing device has no gas permeability, the culturing liquid is optimized for the $CO_2$ concentration before use. In this case, the fertilized egg can be cultured in a completely hermetically sealed container. As long as the temperature can be controlled, the fertilized egg can be cultured anywhere.

The cultured product after the culturing can be recovered by opening the frozen egg culturing device.

According to the frozen egg culturing device of the present invention, it is possible to perform the process of from thawing to culturing in the same device, and obtain blastocysts from frozen eggs. Also, it is confirmed that the blastocysts obtained by the present invention can produce a litter when transplanted.

<Other steps>

The other steps are not particularly limited and may be appropriately selected in accordance with the intended purpose, as long as the other steps do not impair the effects of the present invention. Examples of the other steps include a freezing step and an immobilizing step.

<<Freezing step>>

The freezing step is freezing a fertilized or unfertilized egg in the frozen egg storing container.

A method of freezing the fertilized or unfertilized egg is not particularly limited and may be appropriately selected from publicly known methods in accordance with the intended purpose. Examples of the method include immersing a fertilized or unfertilized egg in a freezing liquid having a low osmotic pressure, and then transferring the fertilized or unfertilized egg to the frozen egg storing container containing a freezing liquid having a high osmotic pressure and rapidly freezing the fertilized or unfertilized egg in liquid nitrogen.

The frozen egg storing container that stores the frozen egg is housed in the housing portion through the opening of the frozen egg culturing device, followed by sealing the opening through heat sealing or the like, to be able to produce the frozen egg culturing device that houses a frozen egg-containing storing container in the housing portion.

The frozen egg culturing device may be stored as is or may be stored in the cooling container.

The temperature of the storage is not particularly limited and may be appropriately selected in accordance with, for example, the kind of the freezing liquid. For example, the temperature may be between the temperature of liquid nitrogen and -30°C.

<<Immobilizing step>>

The immobilizing step is chemically immobilizing the cultured embryo.

The immobilizing step is not a particularly necessary treatment in the fields of fertility treatments and animal husbandry. Meanwhile, when space experiments for detailed observation in basic biology are performed, the embryo needs storing for a long period of time and thus immobilizing.

A method of the immobilization is not particularly limited and may be appropriately selected in accordance with the intended purpose. Considering the space experiments, the embryo is preferably immobilized with paraformaldehyde of lower than 1%.

The immobilization can be performed in the frozen egg culturing device in the same manner as in the above thawing step and the washing step. After the immobilizing step, a washing treatment is optionally performed, and can be stored in a refrigerator or freezer for a long period of time. Also, there has been a risk that a toxic immobilizing liquid would enter the mouth because the chemical immobilization has been conventionally performed using a mouthpiece or the like. The frozen egg culturing device of the present invention, however, does not involve such a risk, having excellent safety.

[Examples]

The present invention will be described below by way of Production Examples and Examples. The present invention should not be construed as being limited to the Production Examples and Examples.

(Production Example 1: Production of frozen egg culturing device)

FROZEBAG F-050 (volume: 250 mL, NIPRO CORPORATION) was modified to produce a frozen egg culturing device. A schematic view of the frozen egg culturing device (reference numeral: 1) is illustrated in FIG. 2A and FIG. 2B.

FIG. 2A is a schematic view illustrating one side of the frozen egg culturing device and FIG. 2B is a schematic view illustrating the other side of the frozen egg culturing device. In FIG. 2A and FIG. 2B, reference numeral 2 denotes a housing portion configured to house a frozen egg contained in a container, reference numeral 3 denotes a liquid injecting portion, reference numeral 4 denotes a liquid discharging portion, and reference numeral 5 denotes an egg outflow preventing portion.

The liquid injecting portion and the liquid discharging portion are ports provided at the end of FROZEBAG F-050.

As the egg outflow preventing portion, a nylon mesh (330 mesh, opening: 40 μm, AS ONE Corporation), which had been cut to a size of 40 mm in length and 50 mm in width, was folded in two at a position 25 mm in the longer direction. The lateral ends of the resulting product were fixed to FROZEBAG F-050 using a heat sealer (P-300, FUJIIMPULSE CO., LTD.). The nylon mesh in FROZEBAG F-050 was disposed in the form of a wall. Specifically, the nylon mesh was fixed, in the longer direction, at a position of 25 mm to 50 mm from the opposite end to the end at which the ports of FROZEBAG F-050 were provided. Meanwhile, the nylon mesh was fixed, in the shorter direction, at a position of 5 mm from the lateral ends of FROZEBAG F-050. The nylon mesh folded in two was placed such that the open side thereof would face the opposite side to the liquid injecting portion and the liquid discharging portion. The nylon mesh does not allow permeation of fertilized and unfertilized eggs, but allows permeation of liquids such as a freezing liquid, a thawing solution, and a culturing liquid.

(Production Example 2: Production of frozen egg storing container)

The upper part of a cryotube (SERUM TUBE, Sumitomo Bakelite Co., Ltd., MS-4501 1 mL) was cut to produce a frozen egg storing container consisting of a bottom part having a shape of letter V in a front view thereof. FIG. 3 is a photograph of the frozen egg storing container.

(Production Example 3: Production of adhesion preventing unit)

An adhesion preventing unit was produced from a cryotube in the following manner.

Specifically, the cryotube was cut, in the vertical direction (the depth direction), into substantially equal five pieces including the respective side surfaces of the cryotube. Each of the cut pieces was further cut into halves to produce curved adhesion preventing units that were each from about 3 mm to about 5 mm in width and about 10 mm in length. Note that, the adhesion preventing unit was subjected to a chamfering process.

(Example 1: Freezing, thawing, washing, and culturing of fertilized egg)

In the present experiment, freezing and thawing of a mouse fertilized egg were performed with reference to the high-osmotic-pressure vitrification method.

<Preparation of mouse fertilized egg>

The mouse fertilized egg used in the present experiment was a fallopian tube perfusion embryo at the two-cell stage that was recovered through fallopian tube perfusion in the following manner.

-Egg recovery through fallopian tube perfusion-

As mice, male and female ICR or B6D2F1 mice were used.

Using a 1 mL syringe (Terumo Corporation) and a syringe needle [26 gauge] (Terumo Corporation), 7.5 IU of PMSG (SEROTROPIN, ASKA Animal Health Co., Ltd.) was intraperitoneally injected to the mice. About 48 hours later, using a syringe in the same manner, 7.5 IU of hCG (GONATROPIN, ASKA Animal Health Co., Ltd.) was injected to perform a superovulation treatment to the female mice.

The female mice, which had undergone the superovulation treatment by the injections of hormones, and the male mice were placed and bred in the same cage overnight. On the next morning, a plug (vaginal plug) was checked. The female mice having the plug were used on the next day.

The mice were euthanized through dislocation of the cervical spine, and the fallopian tube was recovered. The perfusion was performed by pricking an injection syringe (30 gauge) to the fimbria ovarica (the inlet of the fallopian tube on the ovary side), followed by flowing about 0.1 mL of the Hepes-CZB medium (see below; hereinafter this medium may be referred to as a "H-CZB medium"). The fertilized egg recovered through the perfusion was cultured in a chamber containing a CZB medium (see below) coated with mineral oil (M8410, Sigma Co.). The culturing was performed in an incubator (5%$CO_2$, 37°C, 100% humidity).

[CZB medium]

200 mL of Milli-Q was placed in a glass bottle, in which the following reagents were dissolved well using a stirrer, to prepare a stock solution containing the following compounds in the respective amounts. The stock solution was left to stand still in a refrigerator overnight and was sterilized using a filter.

-Compounds contained in stock solution and amounts thereof-

The following amounts represent those per 100 mL of the CZB medium.

| | |
|---|---|
| - NaCl (FUJIFILM Wako Pure Chemical Corporation) | 476 mg |
| - KCl (FUJIFILM Wako Pure Chemical Corporation) | 36 mg |
| - $MgSO_4 \cdot 7H_2O$ (NACALAI TESQUE, INC.) | 29 mg |
| - $KH_2PO_4$ (FUJIFILM Wako Pure Chemical Corporation) | 16 mg |
| - $EDTA \cdot 2Na$ (FUJIFILM Wako Pure Chemical Corporation) | 4 mg |
| - $NaHCO_3$ (FUJIFILM Wako Pure Chemical Corporation) | 211 mg |
| - Sodium lactate (60% syrup) (Sigma Co.) | 0.53 mL |
| - D-glucose (FUJIFILM Wako Pure Chemical Corporation) | 100 mg |
| - Penicillin G (ICN Biomedicals Inc) | 5 mg |
| - Streptomycin (ICN Biomedicals Inc) | 7 mg |
| - Phenol red (10 mg/ml soln. in saline) (Sigma Co.) | Appropriate amount |

The following reagents were added to 50 mL of the stock solution of the CZB medium, and the resulting mixture was left to stand still in a refrigerator overnight.
The following amounts represent those per 100 mL of the CZB medium.

| | |
|---|---|
| - Sodium pyruvate (FUJIFILM Wako Pure Chemical Corporation) | 3 mg |
| - L-glutamine (Sigma Co.) | 15 mg |
| - $CaCl_2 \cdot 2H_2O$ (FUJIFILM Wako Pure Chemical Corporation) | 25 mg |
| - BSA (Sigma Co., GIBCO AlbuMAX) | 500 mg |

The mixture was sterilized using a 0.22 $\mu$m filter (MILLEX GV) in a clean bench. The resulting mixture was dispensed to an assist tube or placed in a glass bottle, and was stored in a refrigerator.

[H-CZB medium]

200 mL of Milli-Q was placed in a glass bottle, in which the following reagents were dissolved well using a stirrer, to prepare a stock solution containing the following compounds in the respective amounts. The stock solution was left to stand still in a refrigerator overnight and was sterilized using a filter.

-Compounds contained in stock solution and amounts thereof-

The following amounts represent those per 100 mL of the H-CZB medium.

| | |
|---|---|
| - NaCl (FUJIFILM Wako Pure Chemical Corporation) | 476 mg |
| - KCl (FUJIFILM Wako Pure Chemical Corporation) | 36 mg |
| - $MgSO_4 \cdot 7H_2O$ (NACALAI TESQUE, INC.) | 29 mg |
| - $KH_2PO_4$ (FUJIFILM Wako Pure Chemical Corporation) | 16 mg |
| - $EDTA \cdot 2Na$ (FUJIFILM Wako Pure Chemical Corporation) | 4 mg |
| - $NaHCO_3$ (FUJIFILM Wako Pure Chemical Corporation) | 211 mg |
| - Hepes $\cdot$ Na (basic) (Sigma Co.) | 520 mg |
| - Sodium lactate (60% syrup) (Sigma Co.) | 0.53 mL |
| - D-glucose (FUJIFILM Wako Pure Chemical Corporation) | 100 mg |

(continued)

| | |
|---|---|
| - Penicillin G (ICN Biomedicals Inc) | 5 mg |
| - Streptomycin (ICN Biomedicals Inc) | 7 mg |
| - Phenol red (10 mg/ml soln. in saline) (Sigma Co.) | Appropriate amount |

The following reagents were added to 50 mL of the stock solution of the H-CZB medium, and the resulting mixture was left to stand still in a refrigerator overnight.

The following amounts represent those per 100 mL of the H-CZB medium.

| | |
|---|---|
| - Sodium pyruvate (FUJIFILM Wako Pure Chemical Corporation) | 3 mg |
| - L-glutamine (Sigma Co.) | 15 mg |
| - $CaCl_2 \cdot 2H_2O$ (FUJIFILM Wako Pure Chemical Corporation) | 25 mg |
| - PVA (Sigma Co.) | 10 mg |

The mixture was sterilized using a 0.22 $\mu$m filter (MILLEX GV) in a clean bench. The resulting mixture was dispensed to an assist tube and was stored in a refrigerator.

<Freezing of mouse fertilized egg and encapsulation into frozen egg culturing device>

The mouse fertilized egg in the CZB medium prepared in the above manner and a small amount of a culturing liquid were transferred to 50 $\mu$L of low-osmotic-pressure freezing liquid 1 (see below; hereinafter this liquid may be referred to as "EFS 20 liquid") that had been returned to room temperature.

[Freezing liquid 1]

-FS liquid for EFS 20-

The following reagents and solution were placed in a 50 mL centrifuge tube, followed by mixing well to dissolve the reagents. After the reagents had been completely dissolved, 60.0 mg of BSA was placed on the top of the resulting solution. After waiting until spontaneous dissolution, the resulting solution was used as FS liquid for EFS 20 liquid. Note that, the compounds contained in PB1 (BSA⁻), the amounts thereof, etc. were described below.

| | |
|---|---|
| - PB1 (BSA⁻)* | 14.0 mL |
| - FICOLL (GE Healthcare) | 6.0 g |
| - Sucrose (FUJIFILM Wako Pure Chemical Corporation) | 3.4 g |

-EFS 20 liquid-

Ethylene glycol was mixed with FS liquid for EFS 20 at the following ratio, to prepare EFS 20 liquid.

| | |
|---|---|
| - FS liquid for EFS 20 | 4 (v/v) |
| - Ethylene glycol (FUJIFILM Wako Pure Chemical Corporation) | 1 (v/v) |

The fertilized egg was transferred to freezing liquid 1. After two minutes had passed, the fertilized egg was transferred to the frozen egg storing container of Production Example 2 containing 50 $\mu$L of high-osmotic-pressure freezing liquid 2 (see below; hereinafter this liquid may be referred to as "EFS 42.5c-d liquid") that had been returned to room temperature.

[Freezing liquid 2]

-FS liquid for EFS 42.5c-d-

The following reagents and solution were placed in a 50 mL centrifuge tube, followed by mixing well to dissolve the reagents. The solution was left to stand still at 4°C in a refrigerator overnight, and was used as FS liquid for EFS 42.5c-d. Note that, the compounds contained in PB1 (BSA⁻), the amounts thereof, etc. were described below.

| | |
|---|---|
| - PB1 (BSA⁻)* | 9.0 mL |
| - FICOLL (GE Healthcare) | 6.0 g |
| - Sucrose (FUJIFILM Wako Pure Chemical Corporation) | 12.0 g |

-EFS 42.5c-d liquid-

Ethylene glycol was mixed with FS liquid for EFS 42.5c-d at the following ratio, to prepare EFS 42.5c-d liquid.

| | |
|---|---|
| - FS liquid for EFS 42.5c-d | 57.5 (v/v) |
| - Ethylene glycol (FUJIFILM Wako Pure Chemical Corporation) | 42.5 (v/v) |

The fertilized egg was transferred to freezing liquid 2. After one minute had passed, the frozen egg storing container was grasped with tweezers and charged to a dish filled with liquid nitrogen. The frozen egg storing container was retained as it was for from 5 to 10 seconds for rapid freezing.

The liquid injecting portion or the liquid discharging portion of the frozen egg culturing device was marked and perforated with a tube. The frozen egg culturing device was cooled in the gas phase of liquid nitrogen. Subsequently, the frozen egg storing container housing the frozen fertilized egg was placed in a housing portion of the frozen egg culturing device. Then, the open portion of the frozen egg culturing device (the opposite end to the end at which the liquid injecting portion and the liquid discharging portion were provided) was sealed with a heat sealer (FUJIIMPULSE CO., LTD., T-130K, T230K) for packing, followed by cooling in liquid nitrogen.

The frozen egg culturing device was frozen in liquid nitrogen and then stored at -80°C.

Note that, the compounds contained the above PB1(BSA-) and PB1(BSA+), the amounts thereof, etc. are as follows.

[PB1(BSA-/+)]

(1) Ten PBS tablets (Takara Bio Inc.) were placed in a screw-cap bottle. 1,000 mL of Milli-Q was weighed and charged to the screw-cap bottle, followed by dissolving and sterilizing through autoclaving. Milli-Q was added to the resulting solution using a graduated cylinder so as to read 1,000 mL. Thereby, 1,000 mL of PBS⁻ was prepared and returned to the screw-cap bottle.

(2) 10 mL of $MgCl_2 \cdot 6H_2O$ (FUJIFILM Wako Pure Chemical Corporation) that had been adjusted to ×100 concentration was dissolved in 1,000 mL of PBS⁻ that had been prepared in the above (1). Once $MgCl_2 \cdot 6H_2O$ had been completely dissolved, 10 mL of $CaCl_2 \cdot 2H_2O$ (FUJIFILM Wako Pure Chemical Corporation) that had been adjusted to ×100 concentration was dissolved in the resulting solution, to prepare 1,020 mL of PBS⁺.

(3) The following reagents were dissolved well in 1,020 mL of PBS⁺ that had been prepared in the above (2), to prepare PB1(BSA⁻).

<Compounds contained in PB1(BSA⁻) and amounts thereof (per 80 mL)>

| | |
|---|---|
| - PBS⁺ | 80 mL |
| - Sodium pyruvate (FUJIFILM Wako Pure Chemical Corporation) | 2.9 mg |
| - Penicillin G (ICN Biomedicals Inc) | 5.1 mg |
| - D-Glucose (FUJIFILM Wako Pure Chemical Corporation) | 80 mg |

(4) 400 mL of PB1(BSA⁻) that had been prepared in the above (3) was dispensed to a 500 mL storage bottle (Corning Inc.). 1,200 mg of BSA (AlbuMAX) was placed on the surface of PB1(BSA⁻) and was left to stand still in a refrigerator until spontaneous dissolution. Then, the resulting solution was sterilized using a filter, to prepare PB1(BSA+).

<Thawing, washing, and culturing of frozen fertilized egg>

-Preparation-

Three 30 mL syringes were provided. The three syringes were charged with 30 mL each of thawing solution 1 (high-osmotic-pressure thawing solution; see below), thawing solution 2 (low-osmotic-pressure thawing solution; see below), and the culturing liquid (the CZB medium), each of which had been returned to room temperature.
Note that, the culturing liquid was, before use, placed in a bag of *Caenorhabditis elegans* having gas permeability, left to stand still for 24 hours in an incubator (5%$CO_2$, 37°C, humidity 100%), and optimized for the $CO_2$ concentration in the culture solution (from 4% to 5%).

[Thawing solution 1]

(1) In a 1,000 mL storage bottle (Corning Inc.), 179.7 g of sucrose was dissolved in PB1(BSA-) under shaking, so that the total amount was adjusted to read a scale of 700 mL of the storage bottle.
(2) 2,100 mg of BSA was placed on the surface of 700 mL of 0.75 M sucrose-PB1 that had been prepared in the above (1) and was left to stand still in a refrigerator until spontaneous dissolution. Then, the resulting solution was sterilized using a filter, to prepare thawing solution 1 (hereinafter may be referred to as "0.75 M sucrose-PB1(BSA+)").

[Thawing solution 2]

200 mL of 0.75 M sucrose-PB1(BSA+) was mixed with 400 mL of PB1(BSA+). Then, the resulting solution was sterilized using a filter, to prepare thawing solution 2 (hereinafter may be referred to as "0.25 M sucrose-PB1(BSA+)").
A waste liquid syringe (100 mL) was attached with a needle-equipped tube configured to connect to the liquid discharging portion of the frozen egg culturing device.
A three-way stopcock was provided. The three-way stopcock was attached with a needle-equipped tube configured to connect to the liquid injecting portion of the frozen egg culturing device. Sequentially from the opposite side of the three-way stopcock to the side thereof at which the needle-equipped tube was attached, a syringe containing thawing solution 1, a syringe containing thawing solution 2, and a syringe containing a culturing liquid were attached.

-Thawing and Washing-

(1) The frozen egg culturing device stored at -80°C was taken out from a freezer.

A tube connected to the waste liquid syringe was mounted to the liquid discharging portion of the frozen egg culturing device, and a tube connected to the three-way stopcock was mounted to the liquid injecting portion of the frozen egg culturing device (see FIG. 4).
The cock of the tube connected to the waste liquid syringe was opened. After the air had been removed with the syringe, the cock was closed.
The present step (1) was performed for 1 minute and 30 seconds at normal temperature. (2) The total amount (30 mL) of thawing solution 1 was injected to the housing portion of the frozen egg culturing device, followed by closing the cock. Then, the frozen egg culturing device was shaken five times.
The present step (2) was performed for 4 minutes and 30 seconds at normal temperature.
(3) The cock of the tube connected to the waste liquid syringe was opened. After the total amount of the injected thawing solution 1 had been removed, the cock was closed.
15 mL of thawing solution 2 was injected to the housing portion of the frozen egg culturing device, followed by closing the cock. Then, the frozen egg culturing device was shaken five times.
The cock of the tube connected to the waste liquid syringe was opened. After the total amount of the injected thawing solution 2 had been removed, the cock was closed.
Next, 15 mL of thawing solution 2 was injected to the housing portion of the frozen egg culturing device, followed by closing the cock. Then, the frozen egg culturing device was shaken five times.
The present step (3) was performed for 3 minutes at room temperature.
(4) The cock of the tube connected to the waste liquid syringe was opened. After the total amount of the injected thawing solution 2 had been removed, the cock was closed.
15 mL of the culturing liquid was injected to the housing portion of the frozen egg culturing device, followed by closing the cock. Then, the frozen egg culturing device was shaken five times.

The cock of the tube connected to the waste liquid syringe was opened. After the total amount of the culturing liquid had been removed, the cock was closed.

15 mL of the culturing liquid was injected to the housing portion of the frozen egg culturing device, followed by closing the cock. Then, the frozen egg culturing device was shaken five times.

(5) After the step (4), the frozen egg culturing device was left to stand still at room temperature (25°C) or 37°C (hereinafter may be referred to as a "temperature until the second medium change") for 0.5 hours, 1 hour, or 2 hours (hereinafter may be referred to as a "duration until the second medium change"). Note that, while the frozen egg culturing device was left to stand still, the frozen egg culturing device was covered with an aluminum foil. Then, the step (4) was repeated once again.

-Culturing-

After the thawing and washing, the tube connected to the three-way stopcock was gradually removed while holding the hard part of the port of the liquid injecting portion. Then, the liquid injecting portion was closed with a rubber cap.

Similarly, the tube connected to the liquid discharging portion was gradually removed, and the liquid discharging portion was closed with a rubber cap.

Next, the frozen egg culturing device was left to stand still in an incubator of 37°C, followed by culturing for 3 days.

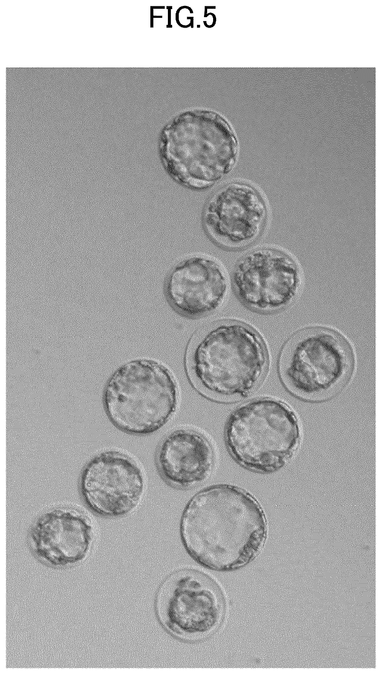

After culturing, the frozen egg culturing device was opened, and the contents were transferred to a 10 cm dish. The rate of blastocysts recovered therefrom and other rates were calculated. The results are presented in Table 1 below. Also, one example of the result obtained through microscopic observation was presented in FIG. 5.

Note that, the recovery rate, the survival rate, and the blastocyst rate were calculated from the following formulae.

$$\text{Recovery rate (\%)} = (\text{Number of embryos recovered/Number of eggs tested}) \times 100$$

$$\text{Survival rate (\%)} = (\text{Number of embryos survived/Number of embryos recovered}) \times 100$$

$$\text{Blastocyst rate (\%)} = (\text{Number of blastocysts/Number of embryos recovered}) \times 100$$

[Table 1]

| Duration until the second medium change | Temperature until the second medium change | Number of eggs tested | Number of embryos recovered (Recovery rate (%)) | Number of embryos survived (Survival rate (%)) | Number of blastocysts (Blastocyst rate (%)) |
|---|---|---|---|---|---|
| 0.5 hours | 37°C | 135 | 91(67) | 47(52) | 45(49) |
| 1 hour | 37°C | 98 | 61(62) | 51(84) | 30(49) |
| 2 hours | 37°C | 106 | 85(80) | 17(20) | 17(20) |
| 0.5 hours | Room temperature | 105 | 82(78) | 33(40) | 33(40) |

As presented in Table 1 and FIG. 5, it was confirmed that by using the frozen egg culturing device of the present invention, culturing from the frozen eggs to blastocysts could be performed.

Also, ICR mice were used as recipients, and the obtained embryos at the blastocyst stage were transplanted into the uteri of pseudopregnant mice on Day 2.5. As a result, litters could be produced (see Table 2), indicating that the blastocysts obtained by culturing in the frozen egg culturing device of the present invention had a quality high enough to be able to produce litters.

[Table 2]

| No. | Number of embryos transplanted | Litter size (Litter rate (%)) |
|---|---|---|
| 1 | 16 | 2 (13) |
| 2 | 16 | 8 (50) |
| 3 | 18 | 6 (33) |
| 4 | 18 | 5 (28) |

Note that, in the fields of fertility treatments and animal husbandry, there is no need to immobilize the cultured embryo. When experiments are performed in a space station, however, there is a need to immobilize the cultured embryo in terms of storage. Also, in a space station, it is preferable to use paraformaldehyde (hereinafter may be referred to as "PFA") of lower than 1% in terms of handling.

In view thereof, the frozen egg culturing device after culturing for 4 days was charged with 0.99% PFA to perform an immobilizing treatment for 1 hour, followed by washing with PBS containing 0.1% polyvinyl alcohol. Note that, injection and discharge of an immobilizing liquid, and injection of a washing liquid were performed in the same manner as in the thawing and washing described above.

The frozen egg culturing device was opened to recover blastocysts.

When the recovered blastocysts were subjected to immunostaining by a routine method, the inner cell mass (ICM) and the trophectoderm (TE) could be stained clearly separately. In addition, when the next-generation sequencer was used to confirm gene expression, it was possible to confirm the expression of Pttg1, a gene necessary for the M phase of a cell cycle.

(Example 2)

A frozen egg culturing device having no adhesion preventing unit was compared with a frozen egg culturing device having an adhesion preventing unit.

<Example 2-1: Frozen egg culturing device having no adhesion preventing unit (control)>

Culturing from frozen eggs to blastocysts was performed in the same manner as in Example 1, except that the temperature and duration until the second medium change in step (5) of -Thawing and Washing- were changed to $37\pm1°C$ and from 1 to 2 hours, respectively.

<Example 2-2: Frozen egg culturing device having adhesion preventing unit>

Culturing from frozen eggs to blastocysts was performed in the same manner as in Example 1, except that: 10 adhesion preventing units each produced in Production Example 3, as well as the frozen egg storing container were placed in the housing portion of a frozen egg culturing container in <Freezing of mouse fertilized egg and encapsulation into frozen egg culturing device> of Example 1; and the temperature and duration until the second medium change in step (5) of -Thawing and Washing- were changed to $37\pm1°C$ and from 1 to 2 hours, respectively.

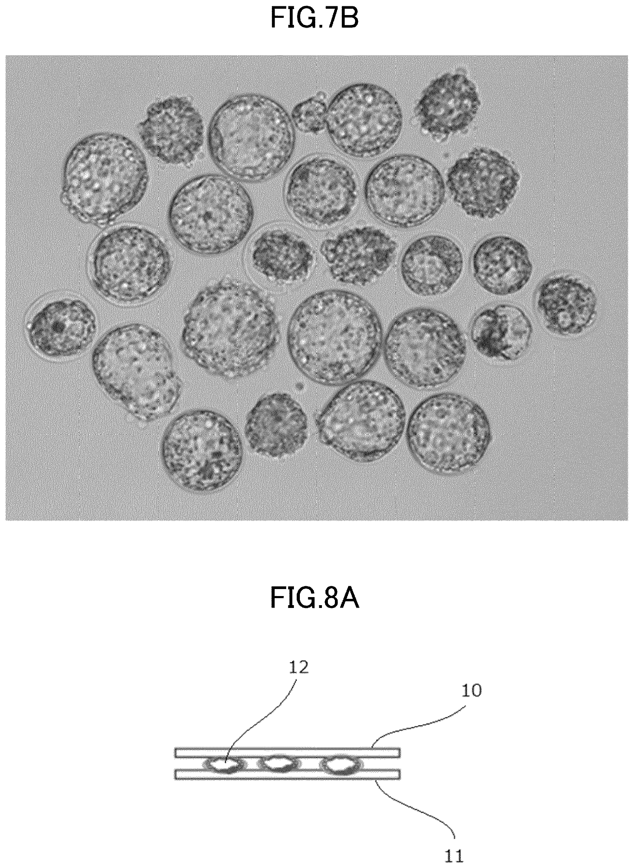

The recovery rates and the blastocyst rates were calculated in the same manner as in Example 1 and presented in Table 2 below. Also, one example of the result obtained through microscopic observation was presented in FIG. 7A (the case of using the frozen egg culturing device having no adhesion preventing unit) or FIG. 7B (the case of using the frozen egg culturing device having the adhesion preventing unit).

[Table 2]

| Example Number | Adhesion preventing unit | Number of eggs tested | Number of embryos recovered (Recovery rate (%)) | Number of blastocysts (Blastocyst rate (%)) | Number and rate of uncollapsed blastocysts (%) | Number and rate of collapsed blastocysts (%) |
|---|---|---|---|---|---|---|
| 2-1 | Absent | 44 | 18 (41) | 18 (18) | 5 (28) | 13 (72) |
| 2-2 | Present | 44 | 40 (91) | 30 (75) | 25 (83) | 5 (17) |

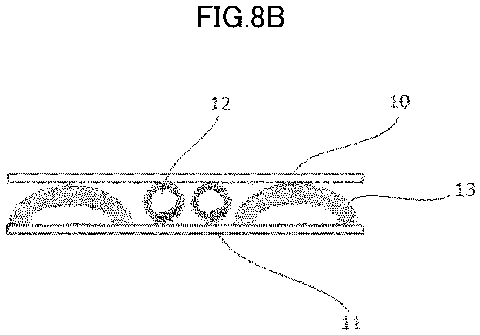

As illustrated in Table 2 and FIGS. 7A and 7B, it was confirmed that by using the frozen egg culturing device having the adhesion preventing units, it was possible to increase the recovery rate of embryos and prevent deformation of embryos. As schematically illustrated in FIGS. 8A and 8B, by providing an adhesion preventing unit (13), it was possible to keep the space in the housing portion upon discharging of, for example, a culturing liquid, to thereby prevent adhesion between an upper surface (10) and a lower surface (11) of the housing portion. Conceivably, this made it possible to prevent deformation of an embryo (12).

According to the frozen egg culturing device of the present invention, the frozen egg can be thawed, washed, and cultured without being directly contacted. The frozen egg culturing device, therefore, enables anyone who has never experienced embryo manipulation to culture frozen eggs without receiving any training. Also, since the frozen egg culturing device is a hermetically closed system, frozen eggs can be cultured anywhere as long as the temperature is controlled.

Thus, the frozen egg culturing device of the present invention can be suitably used in institutions that handle fertilized or unfertilized eggs, such as clinics for fertility treatments, facilities for animal experiments, and animal husbandry. Moreover, the frozen egg culturing device makes it possible to conduct experiments that have been impossible in space.

Aspects of the present invention are, for example, as follows.

<1> A frozen egg culturing device, including:

a housing portion configured to house a frozen egg contained in a container;
a liquid injecting portion;
a liquid discharging portion; and
an egg outflow preventing portion,
the egg outflow preventing portion being between

the frozen egg contained in the container, and
the liquid injecting portion and the liquid discharging portion.

<2> The frozen egg culturing device according to <1> above, wherein the egg outflow preventing portion is placed in the housing portion, and partitions the side of the housing portion at which the frozen egg contained in the container is placed and the side of the liquid injecting portion and the liquid discharging portion.
<3> The frozen egg culturing device according to <1> or <2> above, wherein the container of the frozen egg contained in the container has a shape of letter V in a front view thereof.
<4> The frozen egg culturing device according to any one of <1> to <3> above, wherein the frozen egg is a frozen mammalian egg.
<5> The frozen egg culturing device according to any one of <1> to <4> above, further including an adhesion preventing unit in the housing portion.
<6> A frozen egg culturing method, wherein the frozen egg culturing method uses the frozen egg culturing device according to any one of <1> to <5> above.
<7> The frozen egg culturing method according to <6> above, wherein the frozen egg culturing method includes:

thawing a frozen egg;
washing the thawed egg; and
culturing the washed egg.

<8> The frozen egg culturing method according to <7> above, wherein the washing is performed at least twice.
<9> The frozen egg culturing method according to any one of <6> to <8> above, wherein the frozen egg culturing method uses a culturing liquid having a $CO_2$ concentration of from 3% to 6%.

[Reference Signs List]

1    frozen egg culturing device
2    housing portion
3    liquid injecting portion
4    liquid discharging portion
5    egg outflow preventing portion
6    syringe (for thawing solution 1)

7    syringe (for thawing solution 2)
8    syringe (for culturing liquid)
9    syringe (for waste liquid)
10   upper surface of housing portion
11   lower surface of housing portion
12   embryo
13   adhesion preventing unit

**Claims**

1.  A frozen egg culturing device, comprising:

    a housing portion configured to house a frozen egg contained in a container;
    a liquid injecting portion;
    a liquid discharging portion; and
    an egg outflow preventing portion,
    the egg outflow preventing portion being between

        the frozen egg contained in the container, and
        the liquid injecting portion and the liquid discharging portion.

2.  The frozen egg culturing device according to claim 1, wherein the egg outflow preventing portion is placed in the housing portion, and partitions the side of the housing portion at which the frozen egg contained in the container is placed and the side of the liquid injecting portion and the liquid discharging portion.

3.  The frozen egg culturing device according to claim 1 or 2, wherein the container of the frozen egg contained in the container has a shape of letter V in a front view thereof.

4.  The frozen egg culturing device according to any one of claims 1 to 3, wherein the frozen egg is a frozen mammalian egg.

5.  The frozen egg culturing device according to any one of claims 1 to 4, further comprising an adhesion preventing unit in the housing portion.

6.  A frozen egg culturing method, wherein the frozen egg culturing method uses the frozen egg culturing device according to any one of claims 1 to 5.

7.  The frozen egg culturing method according to claim 6, wherein the frozen egg culturing method comprises:

    thawing a frozen egg;
    washing the thawed egg; and
    culturing the washed egg.

8.  The frozen egg culturing method according to claim 7, wherein the washing is performed at least twice.

9.  The frozen egg culturing method according to any one of claims 6 to 8, wherein the frozen egg culturing method uses a culturing liquid having a $CO_2$ concentration of from 3% to 6%.

# FIG.1

Take out the tube from liquid nitrogen

Add first thawing solution

Transfer the total amount to a dish with a tip

Fertilized eggs are washed while transferred by capillary

Second thawing solution

Transfer to culture solution

LN₂

# FIG.2A

# FIG.2B

FIG.2C

FIG.3

FIG.4

# FIG.5

# FIG.6

a    b    c    d    e

# FIG.7A

# FIG.7B

# FIG.8A

# FIG.8B

## INTERNATIONAL SEARCH REPORT

| International application No. |
| --- |
| **PCT/JP2021/027820** |

| A. | CLASSIFICATION OF SUBJECT MATTER |
| --- | --- |

*C12M 1/00*(2006.01)i; *C12M 3/00*(2006.01)i; *C12N 5/071*(2010.01)i; *C12N 5/073*(2010.01)i
FI:  C12M3/00; C12N5/073; C12N5/071; C12M1/00 A

According to International Patent Classification (IPC) or to both national classification and IPC

| B. | FIELDS SEARCHED |
| --- | --- |

Minimum documentation searched (classification system followed by classification symbols)

C12M1/00-3/00; C12N5/00-5/28

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2009-148218 A (MEIJI UNIV.) 09 July 2009 (2009-07-09)<br>claims, paragraph [0010], fig. 1 | 1-9 |
| A | JP 3226911 U (REPRO SUPPORT MEDICAL RESEARCH CENTER CO., LTD.) 27 July 2020 (2020-07-27)<br>first embodiment, second embodiment, fig. 1-13 | 1-9 |
| A | JP 2020-517269 A (FUJIFILM IRVINE SCIENTIFIC INC.) 18 June 2020 (2020-06-18)<br>paragraphs [0098]-[0101], fig. 11 | 1-9 |
| A | JP 2017-118884 A (DAINIPPON PRINTING CO., LTD.) 06 July 2017 (2017-07-06)<br>claims, fig. 1 | 1-9 |
| A | JP 2010-130999 A (T-CELL TECHNOLOGIES INC.) 17 June 2010 (2010-06-17)<br>claims, fig. 1-17 | 1-9 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 October 2021** | **12 October 2021** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2021/027820**

| Patent document cited in search report | | | Publication date (day/month/year) | Patent family member(s) | Publication date (day/month/year) |
|---|---|---|---|---|---|
| JP | 2009-148218 | A | 09 July 2009 | (Family: none) | |
| JP | 3226911 | U | 27 July 2020 | (Family: none) | |
| JP | 2020-517269 | A | 18 June 2020 | WO 2018/195496 A1 paragraphs [0098]-[0101], fig. 11 | |
| JP | 2017-118884 | A | 06 July 2017 | (Family: none) | |
| JP | 2010-130999 | A | 17 June 2010 | US 2011/0318828 A1 claims, fig. 1-17 | |

Form PCT/ISA/210 (patent family annex) (January 2015)

**REFERENCES CITED IN THE DESCRIPTION**

**Patent documents cited in the description**

- JP 2009148218 A
- JP 2001070402 A
- JP 2010130999 A